Europäisches Patentamt

European Patent Office

Office européen des brevets

(19)

(11) Numéro de publication : **0 162 782**
**B1**

(12) **FASCICULE DE BREVET EUROPÉEN**

(45) Date de publication du fascicule du brevet :
07.03.90

(21) Numéro de dépôt : 85400999.0

(22) Date de dépôt : 21.05.85

(51) Int. Cl.⁵ : **C 12 N 15/00**, C 12 N 5/00,
C 12 N 9/50

(54) Vecteurs d'expression du facteur IX et lignées cellulaires produisant le facteur IX actif.

(30) Priorité : 22.05.84 FR 8407959
05.10.84 FR 8415294

(43) Date de publication de la demande :
27.11.85 Bulletin 85/48

(45) Mention de la délivrance du brevet :
07.03.90 Bulletin 90/10

(84) Etats contractants désignés :
AT BE CH DE FR GB IT LI LU NL SE

(56) Documents cités :
EP-A- 0 083 286
NUCLEID ACIDS RESEARCH, vol. 11, no. 8, avril 1983,
pages 2325-2335, IRL Press Ltd., Oxford, GB; M. JAYE
et al.: "Isolation of a human anti-haemophilic factor
IX cDNA clone using a unique 52-base synthetic
oligonucleotide probe deduced from the amino acid
sequence of bovine factor IX"
NATURE, vol. 302, no. 5908, avril 1983, pages 490-495,
Macmillan Journals Ltd., Chesham, Bucks., GB; G.L.
SMITH et al.: "Infectious vaccinia virus recombinants
that express hepatitis B virus surface antigen"

(73) Titulaire : **TRANSGENE S.A.**
**16, rue Henri-Régnault**
**F-92411 Courbevoie Cédex (FR)**

(72) Inventeur : **de la Salle, Henri**
**10, rue du Général Leclerc**
**F-67400 Ostwald (FR)**
Inventeur : **Drillien, Robert**
**10, rue Paul Deroulède**
**F-67000 Strasbourg (FR)**
Inventeur : **Altenburger, Werner**
**Esterli Weg 135**
**CH-1425 Riehen Canton de B.S. (Bâle) (CH)**
Inventeur : **Toltoshev, Paul**
**28 Barcoo Street**
**East Roseville Sydney (AU)**
Inventeur : **Lecocq, Jean-Pierre**
**6, rue du Champ du Feu**
**F-67460 Reichsteet (FR)**

(74) Mandataire : **Warcoin, Jacques et al**
**Cabinet Régimbeau 26, avenue Kléber**
**F-75116 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

PROCEEDINGS OF THE NATIONAL ACADEMY OF SCIENCES OF THE USA, vol. 80, no. 23, décembre 1983, pages 7155-7159, Washington, US; G.L. SMITH et al.: "Construction and characterization of an infectious vaccinia virus recombinant that expresses the influenza hemagglutinin gene and induces resistance to influenza virus infection in hamsters"

JOURNAL OF VIROLOGY, vol. 49, no. 3, mars 1984, pages 857-864, American Society for Microbiology,; M. MACKETT et al.: "General method for production and selection of infectious vaccinia virus recombinants expressing foreign genes"

CHEMICAL ABSTRACTS, vol. 99, no. 19, 7 novembre 1983, page 179, no. 65367p, Columbus, Ohio, US; & US - A - 445 892 (UNITED STATES DEPT. OF HEALTH AND HUMAN SERVICES) 13-05-1983

CHEMICAL ABSTRACTS, vol. 99, no. 9, 29 août 1983, page 167, no. 153207s, Columbus, Ohio, US; & US - A - 445 451 (UNITED STATES DEPT. OF HEALTH AND HUMAN SERVICES) 13-05-1983

CHEMICAL ABSTRACTS, vol. 72, no. 5, 2 février 1970, page 175, no: 20284k, Columbus, Ohio, US; G.S. RANHOTRA et al.: "Vitamin K and the synthesis of factors VII-X by isolated rat liver cells" & PROC. SOC. EXP. BIOL. MED. 1969, 132(2), 509-13

CHEMICAL ABSTRACTS, vol. 86, no. 7, 14 février 1977, page 314, no. 40873u, Columbus, Ohio, US; H. SUOMELA: "Human coagulation factor IX. Isolation and characterization" & EUR. J. BIOCHEM. 1976, 71(1), 145-54

## Description

La présente invention concerne la construction de lignées cellulaires produisant du facteur IX humain ou des molécules analogues.

Le facteur IX est une protéine intervenant au cours de la coagulation du sang. Il est synthétisé sous forme d'un zymogène et modifié post-traductionnellement par l'addition de chaîne hydrocarbonée, l'hydroxylation d'un acide aspartique et la conversion des 12 acides glutamiques en acides γ-carboxyglutamiques. Cette dernière modification dépend de la vitamine K (Bertina et coll., 1981).

Le foie est le site de synthèse du facteur IX.

Le facteur IX possède une activité réduite ou est absent dans le sang des hémophiles B. L'hémophilie B se manifeste par un temps de saignement très prolongé, comme la forme la plus fréquente de l'hémophilie, l'hémophilie A, et elle est transmise sous forme d'un caractère récessif lié au sexe (Hedner et coll., 1982).

Le facteur IX, lorsqu'il est fourni à des hémophiles B, restaure des temps de saignements normaux.

Pour l'instant, la seule source disponible de facteur IX est le plasma humain, bien que dans certains cas la protéine bovine correspondante puisse être utilisée.

Les préparations de facteur IX peuvent être pyrogènes (provoquer des fièvres) et comportent aussi des risques de contamination des agents pathogènes ou des virus tels que le virus de l'hépatite et les agents vecteurs du S.I.D.A.

C'est pourquoi il est intéressant de développer un procédé permettant la préparation du facteur IX de très haute pureté par la technique des ADN recombinants.

Toutefois, cette technique se heurte à certaines difficultés.

Le facteur IX est une protéine hautement modifiée, ainsi que cela a été mentionné au deuxième paragraphe.

L'importance de ces modifications pour la stabilité ou l'activité du facteur IX est mal connue. On pense que les acides glutamiques modifiés sont essentiels pour l'activation du facteur IX.

C'est pourquoi la Demanderesse a mis au point un vecteur permettant d'exprimer le facteur IX sous une forme active, c'est-à-dire tenant compte des structures particulières évoquées précédemment.

Il s'agit d'un vecteur d'expression constitué par un virus de la famille des Poxvirus, en particulier du genre Orthopoxvirus, par exemple le virus de la vaccine, comportant un gène codant pour le facteur IX humain ou une protéine analogue du facteur IX, ce gène étant sous la dépendance d'un promoteur d'un gène de Poxvirus, par exemple le promoteur du gène codant pour la protéine 7,5K de la vaccine.

Plus particulièrement, la présente invention concerne un procédé de préparation du facteur IX ou d'une protéine de structure analogue, caractérisé en ce que l'on cultive des cellules infectées par un vecteur d'expression du facteur IX constitué du génome d'un virus de la famille des Poxvirus dans lequel a été inséré un gène codant pour le facteur IX humain ou une protéine analogue du facteur IX, et en ce que le milieu de culture comporte de la vitamine K et en ce que l'on récupère, après culture, le facteur IX ou la protéine de structure analogue.

Dans ce qui suit, on parlera essentiellement de virus de la vaccine qui est l'espèce type de la famille et, en particulier, du genre, mais d'autres orthopoxvirus peuvent être utilisés, en particulier le virus Cowpox.

Lorsque l'on indique que le vecteur d'expression est constitué par un virus de la vaccine, il est bien entendu que ce virus peut être incomplet, c'est-à-dire que lors de l'élaboration du vecteur certaines parties peuvent avoir été délétées, dans la mesure où ces délétions ne modifient pas les propriétés essentielles du virus.

De façon générale, on entendra désigner dans la présente description par « protéine analogue au facteur IX » une protéine présentant une analogie structurale et ayant le même type d'activité biologique in vivo ou une activité apparentée, en particulier il peut s'agir d'une protéine ayant la même structure que le facteur IX à certains amino-acides près, des modifications post-traductionnelles comme la glycosylation, la β-hydroxylation et la γ-carboxylation ne sont pas nécessairement identiques à celles de la molécule naturelle, l'important étant d'avoir une molécule présentant une activité coagulante pouvant suppléer celle du facteur IX naturel ; ou bien des fragments du facteur IX ayant l'activité essentielle de la molécule complète.

L'expression d'une séquence codante pour une protéine exogène telle que le facteur IX par le virus de la vaccine (VV) implique nécessairement deux étapes :

1°) La séquence codante doit être alignée avec un promoteur de VV et être insérée dans une région non essentielle de l'ADN de VV, clonée dans un plasmide bactérien approprié.

2°) Les séquences d'ADN de VV situées de part et d'autre doivent permettre des recombinaisons homologues in vivo entre le plasmide et le génome viral. Une double recombinaison réciproque conduit à un transfert de l'insert d'ADN du plasmide dans le génome viral dans lequel il est propagé et exprimé (Panicali et Paoletti, 1982 ; Mackett et coll., 1982 ; Smith et coll., 1983 ; Panicali et coll., 1983).

C'est pourquoi l'ensemble promoteur/séquence codant pour le facteur IX défini précédemment sera inséré dans un gène du virus de la vaccine, par exemple le gène de thymidine kinase (TK), ce qui permet la recombinaison homologue et fournit une possibilité de sélection comme cela sera expliqué ci-après.

Le virus hybride ainsi obtenu peut être utilisé pour infecter une culture cellulaire de laquelle on pourra extraire le facteur IX ou la protéine analogue par des techniques connues à partir des broyats cellulaires ou des milieux de culture.

Les cellules hôtes de vertébré, en particulier de mammifère, peuvent être variées mais adaptées au vecteur mis en œuvre.

En particulier, ces cellules peuvent être des cellules de foie humain qui proviennent donc de l'organe produisant in vivo le facteur IX.

Parmi ces cellules, il faut citer la lignée HepG2. Les procédés permettant de préparer ce type de lignées cellulaires sont décrits notamment dans le brevet WO 81 03663 au nom de The Wistar Institute of Anatomy and Biology.

Ces cellules, bien qu'elles ne fabriquent pas de facteur IX, produisent des protéines de la chaîne de la coagulation sanguine, dont certaines possèdent des propriétés chimiques identiques au facteur IX (facteur X, protéine C, prothrombine). Ces cellules sont donc des hôtes de choix pour la mise en œuvre de la présente invention.

En outre, les expériences effectuées confirment que dans ce procédé d'autres cultures de cellules pouvaient être utilisées, en particulier des cellules de rein telles que les cellules Vero ou les cellules BHK21.

Le virus de la vaccine ne peut accomplir de cycle infectueux sur la lignée CHO. Il n'est donc pas possible d'utiliser le vecteur de la vaccine pour produire une quantité suffisante de facteur IX avec les cellules CHO.

On utilise un vecteur dérivé du virus Cowpox, virus appartenant à la famille du virus de la vaccine, mais capable d'infecter les cellules CHO.

Les cellules transformées ou transfectées sont cultivées sur un milieu approprié assurant leur croissance. Après culture, les cellules ou les milieux de culture sont récoltés et la protéine du facteur IX peut être isolée par des techniques connues de purification des protéines et antigènes.

La présente invention concerne également des procédés de transformation de cellules de mammifère, de culture de ces cellules produisant du facteur IX ou des protéines analogues, ainsi que les protéines analogues au facteur IX obtenues au moins en partie par culture de cellules de mammifère.

De façon plus générale, la présente invention concerne également un procédé de préparation de protéine fabriquée par le foie, caractérisé en ce que l'on cultive une lignée de cellules hépatiques infectée par un virus de la vaccine dans le génome duquel a été inséré le gène codant pour ladite protéine sous la promotion d'un promoteur de la vaccine.

La présente invention a permis, en outre, de mettre en évidence l'importance de la vitamine K dans le milieu de culture cellulaire sur l'activité du facteur IX obtenu.

C'est pourquoi la présente invention concerne plus particulièrement un procédé de préparation du facteur IX actif, caractérisé en ce qu'on cultive une lignée cellulaire de mammifère infectée par un virus recombinant dénommé vecteur d'expression, comme décrit précédemment, la lignée cellulaire infectée étant cultivée sur un milieu comportant de la vitamine K.

La quantité de vitamine K mise en œuvre peut varier en fonction des cultures cellulaires mais sera, de préférence, en quantité assurant la saturation du milieu, par exemple entre 20 et 80 μg/ml de milieu de culture et, en général, sera de l'ordre de 50 μg/ml. Un excès de vitamine K ne semble pas nuisible et cette vitamine est consommée durant la croissance.

Les milieux de culture en eux-mêmes sont connus. Ainsi lorsque les cellules utilisées sont des cellules dérivant de reins telles que les cellules Vero, le milieu utilisé peut être le milieu Dulbecco ; pour les cellules BHK21 le milieu est MEM-Galsgow ; pour les cultures dérivant de foies telles que Chang on utilisera un milieu MEM. Ces milieux sont, de préférence, additionnés de 10 % de sérum de veau fœtal.

D'autres caractéristiques et avantages de la présente invention seront mieux compris à la lecture de ces exemples ci-après.

La préparation du virus vecteur selon l'invention comporte notamment les étapes suivantes :

1) L'obtention d'un clone de cADN correspondant au facteur IX schématisée sur les figures 1 à 3 :

la figure 1 représente le schéma de la sonde de facteur IX avec ses homologues avec le cADN du facteur IX,

la figure 2 représente la séquence d'une partie du cADN obtenu ;

la figure 3 représente la stratégie de préparation de M13tg315.

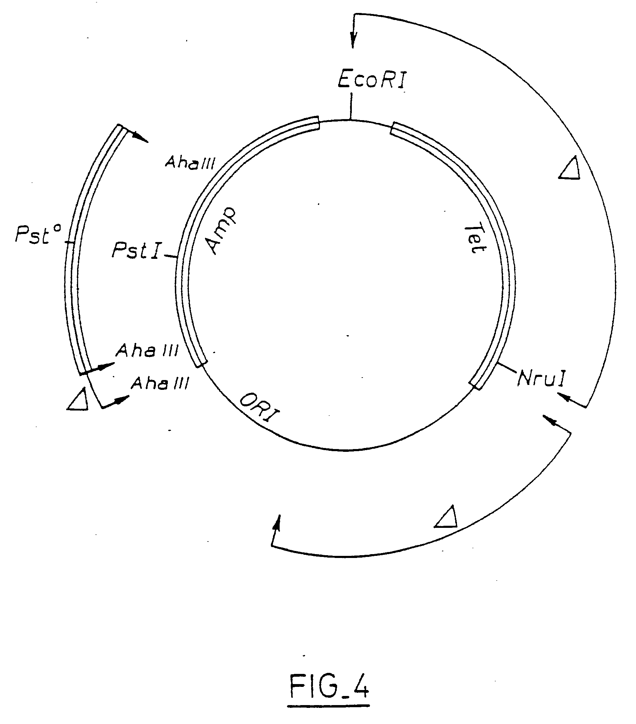

2) La synthèse d'un mini-plasmide pTG1H à partir de pBR322 (figure 4).

3) L'insertion dans ce mini-plasmide du fragment Hin-J portant le gène TK de VV.

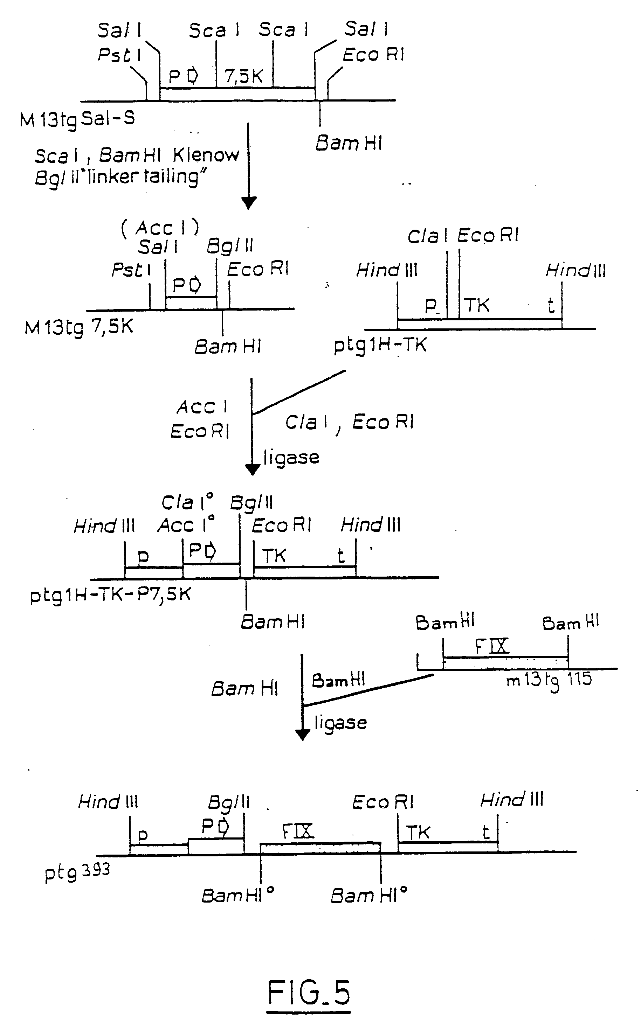

4) L'insertion dans le gène TK du promoteur de la protéine 7,5K (figure 5).

5) L'insertion sous la promotion de P7,5K du gène codant pour le facteur IX porté par M13tg315 (figure 5).

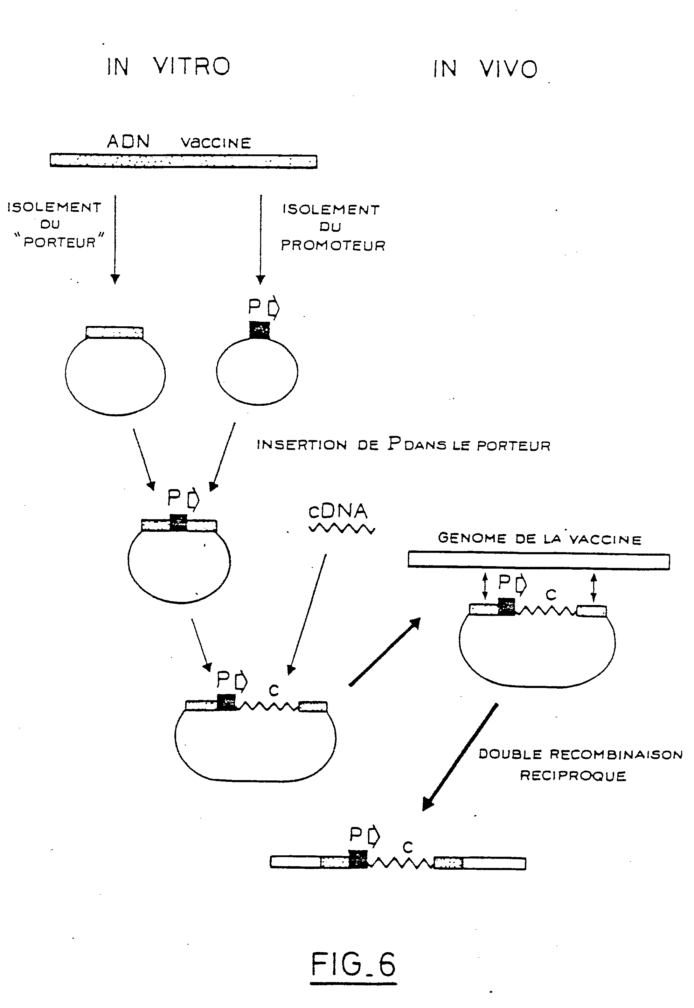

6) Le clonage des éléments essentiels de ce dernier plasmide dans le virus de la vaccine (figure 6).

Les exemples suivants illustrent les propriétés des différents composants obtenus. Les différents matériels mis en œuvre sont identifiés dans les exemples.

Sauf indication contraire, les enzymes sont utilisées dans les conditions préconisées par le fabricant et les techniques mises en œuvre sont connues de l'homme de métier.

Les séquences d'acides aminés et les séquences de nucléotides représentées sur les figures ne sont pas reprises dans le corps de la description pour ne pas l'alourdir mais elles en constituent une partie intégrante.

### Obtention d'un clone de cADN correspondant au facteur IX humain

L'obtention de ce clone de cADN correspondant au facteur IX humain a déjà été décrite dans le brevet français n° 84 07125 au nom de la Demanderesse.

Pour obtenir un clone de cADN correspondant au facteur IX humain, on utilise une sonde constituée d'un oligonucléotide synthétique unique.

De façon surprenante, il a été possible d'obtenir directement le clone du facteur IX à partir d'une banque de cADN de foie humain en utilisant cet oligonucléotide synthétique, bien que seule la séquence d'acide aminé du facteur IX bovin soit connue.

### Matériels et méthodes (Jaye M. et coll.)

#### I. Isolation d'ARN messagers polyadénylés et synthèse du cADN

L'ARN est préparé à partir de 5 g de foie humain en utilisant la technique d'extraction au chlorhydrate de guanidium 8 M, tel qu'il est décrit par Tolstoshev et coll., 1981.

L'ARN ainsi obtenu est chromatographié sur une colonne de polyU-Sepharose (Pharmacia) comme cela est indiqué par le fabricant de façon à obtenir un extrait en ARN contenant du poly-A.

Les séquences de poly-A servent de guide pour la transcription réverse en utilisant des oligo (dT) comme « amorce ». Le cADN a été synthétisé en utilisant 100 $\mu$l de réactif contenant 100 mM de Tris-HCl, pH 8,3, 10 mM de MgCl$_2$, 50 mM de KCl, 30 mM de mercaptoéthanol, 10 $\mu$g/ml d'oligo (dT), 50 $\mu$g/ml de poly-A-ARN, 0,5 mM de chaque de dATP, dGTP, dTTP et dCTP, 80 unités de transcriptase réverse de virus de myoblaste avien (Life Sciences Inc.).

Après 45 minutes à 42 °C, la réaction est terminée et le complexe de cADN-ARN dénaturé par chauffage à 105 °C pendant 3 minutes puis est transféré rapidement sur bain de glace.

Pour la synthèse du second brin d'ADN, le mélange réactionnel précédent est dilué 5 fois et ajusté jusqu'à une concentration finale avec 100 mM HEPES-KOH, pH 6,9, 100 mM de KCl, 200 $\mu$M de dATP, dGTP, dTTP et P32-dCTP (activité spécifique 0,5 Ci/mmole).

10 unités de polymérase ADN de E. coli (fragment Klenow) (Boehringer Mannheim) sont ensuite ajoutées à l'incubation est effectuée à 25 °C pendant 2 heures.

Le cADN double brin (dscADN) est extrait avec un volume égal de phénol : chloroforme (50 : 50), saturé avec 10 mM de Tris-HCl, pH 8, 1 mM d'EDTA, et précipité 2 fois à l'éthanol.

Approximativement 970 ng de dscADN sont obtenus à partir de 5 $\mu$g de poly-A-ARN. Les extrémités sont rendues franches par digestion avec 5 unités de nucléase SI dans un milieu réactionnel de 0,1 ml contenant 30 mM d'acétate de sodium, pH 4,8, 300 mM de NaCl, 3 mM de ZnCl$_2$. Après 1 heure à 37 °C, l'EDTA et SDS sont ajoutés jusqu'à une concentration finale de 10 mM et 0,1 % respectivement et le mélange réactionnel est chauffé à 65 °C pendant 5 minutes.

Le dscADN digéré par SI est alors appliqué sur un gradient de sucrose pré-établi 5-20 % contenant 100 mM de Tris-HCl, pH 7,5, 5 mM d'EDTA, 100 mM de NaCl, et centrifugé à 30 000 tours par minute à 15 °C pendant 16 heures sur un rotor SW60 Ti.

Les fractions de 0,5 ml sont collectées. Pour déterminer la taille du cADN, 1 $\mu$l de chaque fraction est analysé par électrophorèse sur un gel d'agarose neutre et la migration de chaque fraction est comparée avec un marqueur de poids moléculaire approprié.

Les fractions contenant des cADN plus grands que 1 kilobase sont rassemblées puis précipitées avec 2 volumes d'éthanol pendant une nuit en présence de 5 $\mu$g de tARN de E. coli comme entraîneur. Le précipité est remis en suspension dans 20 $\mu$l 0,1 × SSC (5 mM de NaCl, 0,5 mM de citrate de sodium, pH 7,0).

#### II. Clonage du cADN

Des éléments d'extrémités homopolymères (approximativement 15 dCMP) sont ajoutés à l'extrémité 3' du cADN en utilisant la déoxynucléotidyl terminale transférase (Deng et coll., 1981).

De manière similaire, des éléments d'extrémités homopolymères poly-dGMP (approximativement 10 dGMP) sont ajoutés aux extrémités 3' du plasmide pBR322 préalablement digéré avec l'enzyme de restriction PstI.

60 ng de cADN possédant des extrémités de poly-C-dCMP et 1 $\mu$g du vecteur préparé dans les conditions du paragraphe précédent sont chauffés pendant 2 heures à 42 °C dans 10 mM de Tris-HCl, pH 7,5, 100 mM de NaCl. Les plasmides recombinants sont utilisés pour transformer la souche E. coli 8739 (Murray et coll., 1977) et les transformants sont sélectionnés sur plaque d'agar-LB contenant 15 $\mu$g/ml de tétracycline. On obtient 30 000 transformants dont environ 50 % comportent un insert de cADN supérieur à 1 kb. Tous les transformants sont prélevés des plaques dans un volume minimum de LB et après addition d'un volume égal de glycérol, la banque obtenue est stockée à — 20 °C.

5

III. Sélection et synthèse de la sonde oligonucléotide pour le facteur IX

1 755 nucléotides de séquences de gènes codant pour des protéines bovines synthétisées par le foie sont analysés (Mac Gillivray et coll., 1980 et Chung et coll., 1981) de façon à mettre en évidence les codons utilisés préférentiellement. On a sélectionné une séquence de 52 bases. Cet oligonucléotide est construit de la façon suivante :

L'oligonucléotide A : d(CTCACACTGATCACCATCCACATACT), l'oligonucléotide B : d(GCTTCCA-GAACTCTGTAGTCTTCTCA) et le fragment complémentaire C : d(GGAAGCAGTATGT) sont synthétisés chimiquement par la méthode phosphotriester sur un support solide inorganique comme cela a été décrit précédemment par Kohli et coll., 1982 et purifiés par HPLC comme décrit par Fritz, 1978.

0,5 nmole d'oligonucléotide B est mis en incubation pendant 30 minutes à 37 °C dans un milieu réactionnel de 10 μl contenant 60 mM de Tris-HCl, pH 7,8, 6 mM de $MgCl_2$, 6 mM de dithiothreitol, 0,1 mM d'ATP, 6 pmoles P32-ATP (3 000 Ci/mmole), 2 unités de kinase T4 polynucléotide. L'oligomère B 5′ phosphorylé est mélangé avec 0,5 nmole d'oligonucléotide A et C dans 10 mM de Tris-HCl, pH 7,5, 1 mM d'EDTA, chauffé à 100 °C et le mélange est refroidi lentement jusqu'à 4 °C. Le mélange réactionnel des oligonucléotides A et B est ligué dans 50 μl de mélange réactionnel contenant 66 mM de Tris-HCl, pH 7,5, 100 mM de NaCl, 7,5 mM de $MgCl_2$, 2 mM de dithiothréitol, 0,5 mM de spermidine, 0,2 mM d'EDTA, 4 unités de ligase DNA T4 à 4 °C pendant une nuit.

Le 52mer résultant est alors purifié du mélange de ligation par électrophorèse sur gel de polyacrylamide à 20 %.

IV. Analyse de la banque de cADN de foie humain

10 000 colonies environ sont mises en croissance pendant une nuit sur plusieurs boîtes d'agar LB contenant 10 μg/ml de tétracycline. Le jour suivant les colonies bactériennes sont transférées sur filtres de nitrocellulose et les filtres sont préparés pour l'hybridation sur des colonies, essentiellement comme cela a été décrit par Grunstein et coll., 1979. Les bactéries restantes, sur les boîtes à la tétracycline d'origine, sont remises en croissance pendant quelques heures à 37 °C.

La sonde d'hybridation est préparée par kination de 100 ng du 52mer avec 5 μCi de P32-ATP. L'hybridation est effectuée à 48 °C pendant une nuit dans 40 ml de 6 × SSC et 1 X de solution de Denhardt, 0,1 % de SDS, 50 μg/ml d'E. coli tARN.

Le jour suivant, les filtres sont lavés à 42 °C plusieurs fois avec une solution de 6 × SSC, 0,1 % de SDS. Les filtres sont séchés puis autoradiographiés pendant une nuit.

Résultats

La séquence d'acides aminés du facteur IX bovin (Katayama et coll., 1979) est analysée pour trouver une séquence longue composée d'acides aminés déterminés par des codons faiblement dégénérés.

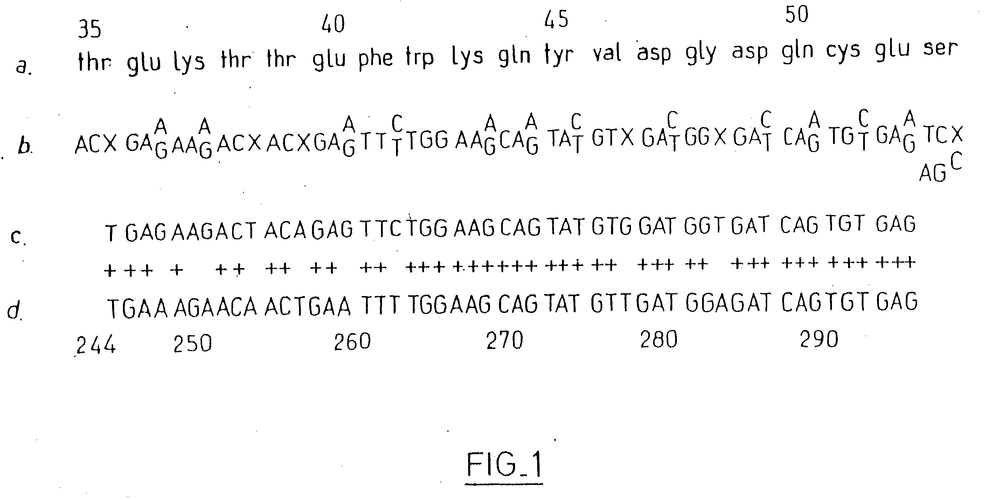

La sonde a ainsi été réalisée pour correspondre aux acides aminés 36 à 52 du facteur IX bovin, qui correspond à 4 acides aminés codés par 4 codons (thr, val, gly), 12 acides aminés codés par 2 codons (glu, lys, phe, gln, tyr, asp, cys) et 1 acide aminé codé par 1 codon (trp) (figure 1).

Ensuite, l'analyse de 585 codons de la prothrombine bovine et du fibrinogène bovin ont révélé que le codon valine GTG est utilisé 21 fois sur 38. De même TTC et TGT sont utilisés 2 fois plus fréquemment que les autres codons des acides aminés phe et cys, respectivement.

Aucune autre préférence très nette ne se détache à l'analyse, toutefois, certains nucléotides sont trouvés peu fréquemment en 3ème position pour certains codons, par exemple G dans le cas de la glycine et de la thréonine.

En outre, l'appariement G : T, bien que moins stable que la paire de bases G : C, contribuera au moins à la stabilisation de l'hybridation du 52mer durant l'hybridation de la colonie, tandis que l'appariement A : C ne le fera pas.

Ainsi, G a été choisi pour chacun des acides aminés (glu, lys, gln) dans lesquels le choix en 3ème position du codon dégénéré était soit G soit A ; de même T a été choisi par rapport à A dans le cas des acides aminés tyr et asp.

Compte tenu de ces considérations, le choix s'est limité à T ou G comme nucléotide en 3ème position pour la thréonine et la glycine ; toutefois, comme cela a été indiqué précédemment, G se trouve peu fréquemment en 3ème position des codons de la thréonine. Ainsi on a choisi T pour l'un des codons de la thréonine et de façon à écarter un problème possible d'une structure secondaire à l'intérieur du 52mer, choisi T en position 3 pour la glycine et A en position 3 pour le second codon de la thréonine.

Sélection de la banque de cADN humain et identification du clone de facteur IX

La stratégie d'origine était d'utiliser le 52mer pour sélectionner la banque de cADN de foie de bovin et ensuite utiliser ce clone de cADN de facteur IX bovin sous forme d'une sonde pour une banque de cADN

EP 0 162 782 B1

de foie humain. Cette stratégie reposait sur une hypothèse de généralisation de l'homologie connue dans les séquences d'acides aminés à l'extrémité N des protéines bovines et humaines (voir Di Scipio et coll., 1977).

En s'appuyant sur cette homologie interspécifique dans les séquences d'acides aminés, on a effectué une sélection parallèle d'une banque de foie de bovin et de foie humain.

Les conditions d'hybridation et de lavage employées furent peu sévères car l'homologie actuelle entre la sonde 52mer et le cADN de facteur IX était inconnue.

Différentes colonies dans la banque de cADN bovin donnèrent des signaux positifs par sélection avec la sonde 52mer. Dans la banque de cADN de foie humain, une colonie a été obtenue, qui donne un signal dans les conditions mises en œuvre. Compte tenu du fait que la présente recherche a été effectuée dans le but d'isoler un cADN de facteur IX humain, il n'a pas été effectué d'autres analyses sur les clones bovins et tous les efforts se sont portés sur le clone humain.

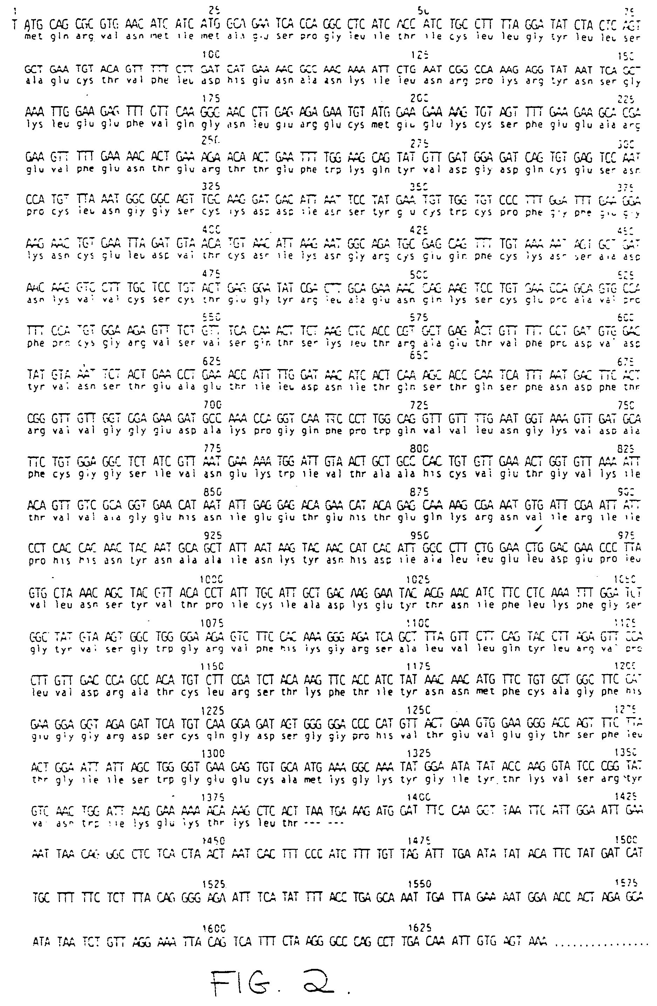

Pour établir que cette colonie correspond au facteur IX humain, le plasmide recombinant est digéré avec PstI, puis l'insert de cADN excisé est transféré dans le phage M13mp8 (Messing et coll., 1982) et séquencé en utilisant des didéoxynucléotides comme terminateurs de chaîne (Sanger et coll., 1977). La séquence obtenue correspond aux nucléotides 1-180 de la figure 2, la séquence d'acides aminés déduite confirme que le clone est un clone de cADN de facteur IX humain.

La séquence plus complète de l'insert de cADN de ce clone, pTG397, est déterminée en combinant les méthodes de Maxam et de Gilbert, 1980, ou de Sanger, à partir de segments de restriction sous clonés dans M12mp9. La séquence obtenue est indiquée dans la figure 2.

Discussion du résultat

En employant une sonde oligonucléotide unique, il a été possible d'isoler un clone de facteur IX humain contenant un insert de 2,1 kb. Lorsque l'insert de 2,1 kb est utilisé comme sonde pour trier une seconde banque de cADN de foie humain, un autre clone spécifique de facteur IX, pTG398, contenant un insert de 2,6 kb est isolé.

L'extrémité 5' de pTG398 est située au nucléotide 480 de la figure 2. Ceci suggère que l'extrémité non traduite 3' de pTG397 est incomplète et que l'extrémité complète 3' non traduite a une longueur d'environ 1,7 kb.

On observe une différence entre la séquence de nucléotide obtenue et la séquence donnée par Kurachi et Davie.

Cette différence comporte une transition au nucléotide 581 (G- >A), transformant l'alanine en thréonine. La détermination de la séquence d'acides aminés du peptide de connexion du facteur IX confirme la présence de thréonine dans cette position. Ces données suggèrent que comme pour d'autres protéines sériques le facteur IX est polymorphe (Cooper, 1978).

Cette hypothèse a été confirmée (MacGran R.A. et al., Blood 64, 264a, 1984).

En considérant l'utilisation des codons de substitution G:T et l'élimination de la structure secondaire possible dans la sonde de nucléotide, on a pu obtenir un 52mer unique qui s'est révélé être utile de façon satisfaisante comme sonde pour le séquençage d'un clone unique. Le 52mer choisi est homologue avec la séquence clonée pour 43/52 nucléotides tandis que deux appariements erronés sont des paires G:T.

Ceci reflète une homologie à 85 % dans les séquences de nucléotides interspécifiques observées précédemment.

Structure générale du cADN de facteur IX

La connaissance des séquences de nucléotide du cADN de facteur IX permet de déduire la séquence d'acides aminés codée de la protéine correspondante. L'insert de cADN de pTG397 révèle les caractéristiques suivantes du facteur IX humain :

a. Le facteur IX est une protéine contenant 415 acides aminés. Dans la figure 2, les nucléotides 140-1384 codent pour le facteur IX humain.

b. Le facteur IX humain est synthétisé sous forme d'un précurseur avec une séquence signal attachée et une séquence de préprotéine. Ceci est une caractéristique commune à toutes les protéines qui sont destinées à être sécrétées. Dans le cas du facteur IX, il y a une séquence signal classique d'environ 25 acides aminés codés par les nucléotides 2-76, inclus. L'extension hydrophobe des acides aminés est normalement éliminée pendant la sécrétion de la protéine à l'extérieur de la cellule. Les nucléotides 77-139 inclus codent pour une séquence de préprotéine qui probablement rapidement après la sécrétion est éliminée de la forme mature du facteur IX (commençant avec la tyrosine au nucléotide 140).

Comme cela est souvent trouvé à la fin de la séquence de préprotéine, la structure d'acides aminés dibasiques lys-arg est trouvée à la jonction entre la séquence de préprotéine et le premier acide aminé du facteur IX mature.

c. La forme activée du facteur IX (IXa) est produite à partir de la forme inactive ou zymogène du facteur IX par hydrolyse de deux liaisons peptides par le facteur XI activé (XIa). Ces liaisons peptides sont trouvées entre les acides aminés arg-ala (nucléotides 572-577) et val-val (nucléotides 677-682). Le peptide le plus long de 35 acides aminés libéré pendant l'activation du facteur IX est appelé le peptide

7

d'activation. Le facteur IXa est ainsi composé de deux chaînes polypeptidiques : une chaîne légère de 145 acides aminés (nucléotides 140-574) et une chaîne lourde de 235 acides aminés (nucléotides 680-1384). Les chaînes légères et lourdes du facteur IXa sont maintenues ensemble par des ponts disulfures. La chaîne lourde comprend un site actif typique de la sérine-protéase (met-phe-cys-ala-gly, nucléotides 1181-1195) de même que d'autres résidus importants dans l'activité catalytique de la sérine-protéase typique. La chaîne légère contient 12 unités d'acide glutamique qui sont γ-carboxylées pour produire l'acide γ-carboxy-glutamique par un processus qui dépend de la vitamine K.

Ceci est important dans la fixation du facteur IXa au Ca$^{++}$, à la membrane phospholipidique membranaire et au facteur VIIIa dans le cycle de coagulation.

d. Au contraire du facteur IX bovin qui a 4 sites pour la fixation d'hydrate de carbone, le facteur IX humain a 2 sites potentiels pour l'attachement des hydrates de carbone. Ces sites sont tous situés dans le peptide d'activation, aux nucléotides 608-616 et 638-646, et contiennent la séquence typique asn-x-thr/ser. Par contraste, le facteur IX bovin a 4 hydrates de carbone attachés dont la structure a été récemment déterminée par Mizurochi et coll., 1983.

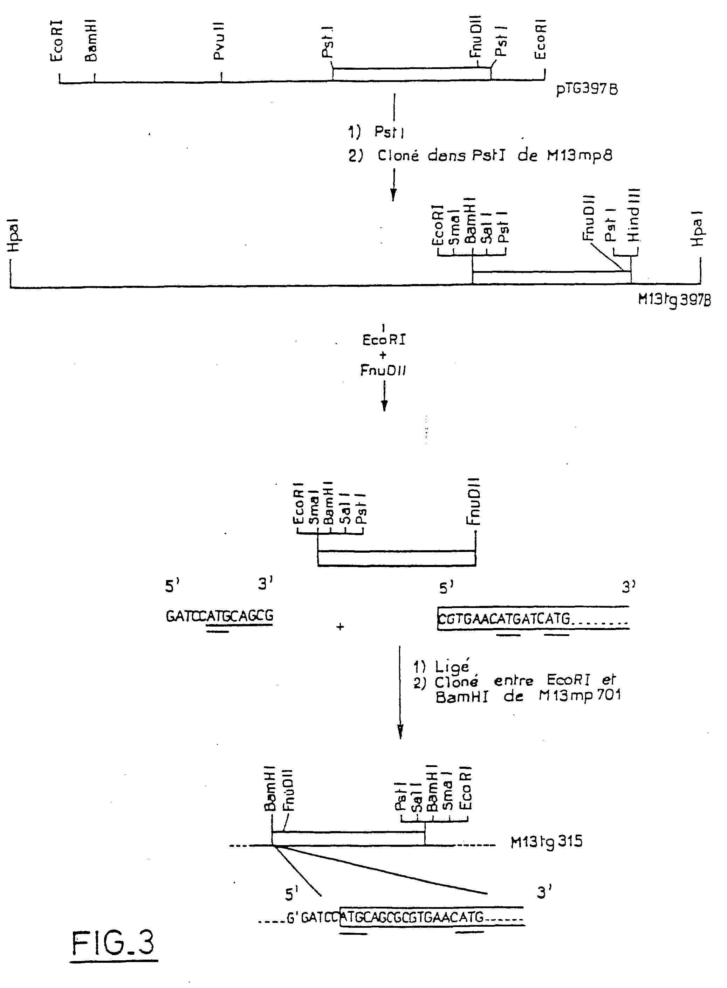

Préparation du cADN de facteur IX cloné nécessaire pour l'expression (voir figure 3)

pTG397 a été mis en digestion avec l'enzyme de restriction PstI. Comme la banque de cADN a été clonée par des extrémités G/C dans le site PstI de pBR322 et, en outre, que le facteur IX de pTG397 ne contient pas un site interne de reconnaissance PstI, ce traitement libère un cADN intact de 2,1 kb correspondant au facteur IX, avec des extrémités susceptibles d'être clonées dans les sites PstI.

Le fragment de cADN facteur IX est transféré par des procédures standards dans le phage M13mp8 qui a été précédemment mis en digestion avec PstI et traité avec la phosphatase alcaline. Les phages recombinants sont utilisés pour transfecter une souche de E. coli JM103.

Le phage recombinant M13tg397B est utilisé comme source de cADN de facteur IX comme cela sera décrit ci-après.

Le cADN de facteur IX contient un site de reconnaissance unique pour l'enzyme FnuDII qui reconnaît la séquence de tétranucléotide CGCG située sur les nucléotides 7-10.

Par digestion de M13tg397B avec PstI et FnuDII, on obtient un grand fragment et de nombreux petits fragments, correspondant à 19 sites de reconnaissance pour FnuDII dans M13mp8. Le fragment le plus grand correspondant au fragment FnuDII:PstI.

Ce fragment écarte 9 nucléotides et les extrémités G/C de l'extrémité 5′ du cADN de facteur IX. Il a été considéré comme important d'écarter les extrémités G/C de l'extrémité 5′ de façon à augmenter l'expression et la stabilité du cADN de facteur IX dans le vecteur d'expression.

En éliminant ces 9 nucléotides, on élimine un ATG de la séquence signal. Il n'est pas sûr toutefois que cet ATG soit l'ATG qui initie la traduction. La portion du facteur IX éliminée par digestion avec FnuDII est dans la séquence signal et en conséquence n'est pas importante pour l'activité du facteur IX.

La séquence signal, si la traduction débute normalement à l'ATG éliminé lors de la digestion par FnuDII, est écourtée de 5 acides aminés. De façon à déterminer si ces 5 acides aminés additionnels sont importants pour la sécrétion et/ou pour l'utilisation du facteur IX, la séquence de nucléotide éliminée par digestion avec FnuDII est restaurée en utilisant deux oligonucléotides synthétiques :

A)   5′   GATCCATGCAGCG   3′
             ------------

B)   5′   CGCTGCATG   3′

La séquence soulignée dans l'oligonucléotide A correspond à la séquence écartée, moins le premier nucléotide T, du phage M13tg397B par digestion avec FnuDII.

L'oligonucléotide B est complémentaire de l'oligonucléotide A.

L'hybridation des oligonucléotides A et B donne la structure à double brin suivante qui possède des extrémités BamHI cohésives :

5′   GATCCATGCAGCG   3′

3′   GTACGTCGC   5′

Les oligonucléotides A et B sont ligués avec le fragment de cADN de facteur IX EcoRI/FnuDII qui a été purifié à partir de M13tg397B. Le phage M13mp701 traité par le phosphatase-alkaline et soumis à la restriction par BamHI est alors ajouté pour la ligation et la ligation est effectuée pendant toute une nuit. Un aliquote du mélange de ligation final est transformé dans une cellule JM103. La séquence de nucléotide du phage recombinant M13tg315 qui a donné une plage blanche confirme que la construction est correcte. La digestion de M13tg315 avec BamHI libère un fragment de cADN de facteur IX dont la séquence 5′ est la suivante :

8

```
              1        10        20
      5'      G GATCCATGCAGCGCGTGAACATG////////FIX////////////G GATCC 3'
              /    ...............
              /    ----------------
          BamHI                                              BamHI
```

La séquence correspondant à l'oligonucléotide A a été indiquée ci-dessus en traits pointillés. La séquence soulignée correspondant aux 15 nucléotides qui codent pour les 5 acides aminés absents de la séquence signal du fragment de cADN de facteur IX BamHI/BglII de M13tg311.

Construction des plasmides hybrides

Les tailles combinées des différents éléments nécessaires pour le transfert de la séquence codant pour le facteur IX dans le génome de VV et son expression subséquente sont de l'ordre de plusieurs kb. Il a donc été jugé nécessaire de minimiser la taille du plasmide de réplication dans E. coli utilisé pour le travail de construction de façon à faciliter les manipulations nécessaires.

Le fragment HindIII (Hin-J) du génome de VV contient le gène complet de la thymidine kinase (TK) qui a déjà été utilisé précédemment pour permettre l'échange et la recombinaison de l'ADN à insérer dans le génome de VV (Mackett et al., 1982). Il est important de noter que le transfert d'un insert dans le gène TK dans le génome de VV crée un virus TK déficient qui peut être reconnu en utilisant une simple sélection. Il a tout d'abord été nécessaire de produire un plasmide de petite taille portant un site unique HindIII utilisable pour l'intégration du fragment Hin-J VV. En outre, il était important d'éliminer les séquences de restriction non nécessaires du plasmide de façon à permettre les manipulations suivantes.

3) La construction a été amorcée à partir du plasmide pML2 (Lusky et Botchan, 1981) qui est un vecteur dérivé du plasmide pBR322 par délétion spontanée du segment entre le nucléotide 1089 et 2491 (figure 4).

D'abord la séquence de PstI a été éliminée par insertion du fragment AhaIII-AhaIII de pUC8 (Vieira et Messing, 1982) entre deux sites AhaIII de pML2 en éliminant 19 paires de bases. On a utilisé la méthode de « linker-tailing » (Lathe et al., 1984) pour insérer un linker HindIII entre les sites NruI et EcoRI traité par la nucléase SI en éliminant le site BamHI. Ceci conduit à un plasmide de 2 049 paires de bases portant le gène β-lactamase fonctionnel (conférant la résistante à l'ampicilline) et comportant en outre une origine de réplication active dans E. coli et un site de restriction unique HindIII.

Cette construction a été appelée pTG1H.

4) Le fragment Hin-J de l'ADN de VV portant le gène TK a préalablement été cloné dans un vecteur provenant de pBR327 (Drillien et Spehner, 1983). Ce fragment de 4,6 kb a été recloné dans le site HindIII de pTG1H. Un clone a été sélectionné dans lequel le gène TK est situé distalement par rapport au gène codant pour la résistance à l'ampicilline.

Cette construction pTG1H-TK a été utilisée comme porteur dans l'expérience suivante.

5) L'étape suivante a été d'isoler un promoteur de VV utilisable pour commander l'expression de la séquence codant pour le facteur IX inséré. Le promoteur d'un gène précoce codant pour une protéine de 7 500 daltons (7,5K) a déjà été utilisé avec succès dans un but identique (Smith et al., 1983) et on a donc procédé à son isolement.

Le gène 7,5K est situé sur l'un des plus petits fragments SalI (fragment Sal-S) du génome de VV type WR (Venkatasan et al., 1981). Comme les petits fragments sont clonés de façon préférentielle, une grande proportion des clones obtenus par clonage direct de l'ADN de VV type WR coupé par SalI dans le plasmide pBR322 porte le fragment Sal-S. Ce fragment est transféré sur le bactériophage vecteur M13mp701 (voir Kieny et al., 1983), par digestion SalI et religation, en conduisant ainsi au phage M13tgSal-S.

Dans ce clone, un site ScaI se trouve immédiatement à proximité de l'ATG d'initiation du gène 7,5K. En aval du gène 7,5K se trouvent situés des sites uniques BamHI et EcoRI provenant du vecteur. Les sites BamHI et ScaI sont fusionnés par l'intermédiaire d'un linker BglII 5'-CAGATCTG-3' par la technique des « linker » après avoir complété les extrémités générées par digestion BamHI avec le fragment Klenow de la polymérase. Ce procédé élimine le site ScaI mais reconstitue le site BamHI et place le site unique EcoRI immédiatement en aval du site BamHI. En même temps, le site SalI(AccI) en aval est éliminé, le site en amont devient donc unique.

Cette construction est appelée M13tg7,5K.

A l'intérieur du fragment Hind-J de l'ADN de VV se trouvent situés des sites ClaI et EcoRI qui sont séparés par environ 30 paires de bases (Weir et Moss, 1983). Le fragment promoteur de 7,5K présent dans M13tg7,5K est excisé par AccI et EcoRI et cloné entre les sites ClaI et EcoRI de pTG1H-TK pour générer pTG1H-TK-P7,5K dont la synthèse est schématisée figure 5.

Cette construction conduit au transfert du site BamHI unique du vecteur M13 immédiatement en aval de la séquence du promoteur 7,5K. Ce site unique BamHI est utilisé dans les constructions suivantes.

9

6) pTG1H-TK-P7,5K est mis en digestion avec BamHI et ligué avec M13tg115 digéré par BamHI (figure 5).

Après transformation de E. coli l'un des plasmides recombinants isolé par cette procédure, pTG393, est sélectionné car il porte le cADN du facteur IX dans l'orientation correcte pour l'expression à partir du promoteur de 7,5K.

Clonage dans le virus de la vaccine (figure 6)

7) La stratégie décrite par Smith et al. (1983) repose sur l'échange in vivo entre un plasmide portant un insert dans le gène VV TK et le génome viral de type sauvage de façon à inactiver le gène TK porté par le virus. Les virus TK⁻ peuvent être sélectionnés par étalement sur une lignée cellulaire TK-négative en présence de 5-bromodéoxyuridine (5BUDR) (Mackett et al., 1982). La thymidine kinase phosphoryle le5BUDR en 5'-monophosphate, qui est ensuite converti en triphosphate. Ce composé est un analogue de dTTP et son incorporation dans l'ADN bloque le développement correct du virus. Un virus TK⁻ peut néanmoins répliquer son ADN normalement et il conduit à des plaques virales visibles dans une couche cellulaire également TK⁻.

Le virus de la vaccine se propage dans le cytoplasme des cellules infectées plutôt que dans leur noyau. C'est pourquoi il n'est pas possible de tirer avantage de la machinerie de réplication et de transcription de l'ADN de l'hôte et il est nécessaire que le virion possède les composants pour l'expression du gène viral. L'ADN de VV purifié est non infectieux.

Afin de générer les recombinants, il est nécessaire d'effectuer simultanément l'infection cellulaire avec un VV et une transfection avec le segment d'ADN cloné qui présente de l'intérêt. Toutefois, la génération des recombinants est limitée à une petite proportion des cellules compétentes pour la transfection à l'ADN. C'est pour cette raison qu'il a été nécessaire de mettre en œuvre une stratégie de « congruence » indirecte pour réduire le bruit de fond des virus parentaux non recombinants. Ceci a été effectué en utilisant comme virus infectieux vivant un mutant thermosensible (ts) de la vaccine qui n'est pas capable de se propager à une température non permissible de 39,5 °C (Drillien et Spehner, 1983). Lorsque les cellules sont infectées avec un mutant ts dans des conditions non permissibles et transfectées avec l'ADN d'un virus de type sauvage, la multiplication virale interviendra seulement dans les cellules qui sont compétentes pour la transfection ; aucun résultat ne sera obtenu dans les autres cellules, en dépit du fait qu'elles ont été infectées. Si un plasmide recombinant contenant un fragment de l'ADN de vaccine tel que pTG393 est inclus dans le mélange de transfection à la concentration appropriée avec l'ADN du type sauvage, il est également possible d'obtenir qu'il participe à la recombinaison homologue avec l'ADN de la vaccine dans les cellules compétentes.

Clonage des recombinants VV-FIX

Des fibroplastes d'embryons de poulets primaires (CEF) établis à partir d'embryons âgés de 11 jours sont mis en croissance jusqu'à confluence ($2 \times 10^6$ cellules/boîte de 2 cm de diamètre) dans un milieu de base de Eagle (MBE/Eurobio) supplémenté avec du sérum de veau nouveau-né (SNV) (Drillien et Spehner, 1983).

Les cellules sont infectées à raison de 0,01 à 0,1 pfu par cellule avec un mutant du virus de la vaccine thermosensible N7 dérivé du virus de type sauvage Copenhague (Drillien et coll., 1981).

Après absorption du virus, les cellules sont incubées à une température permise pour le virus (33 °C) pendant 2 heures dans MBE/SCN 5 %. Les cellules sont alors transfectées avec un mélange d'ADN virus de type sauvage (Wt) Copenhague purifié (30 ng par boîte) et l'ADN du plasmide chimérique contenant le gène étranger (30 ng/boîte) en utilisant la méthode de coprécipitation au calcium-phosphate.

Après 1 heure d'absorption, les cellules sont incubées pendant 2 heures à 39,5 °C en présence de MBE/2 % SCN. Le précipité calcium est lavé 3 fois avec PBS et les cellules sont mises en croissance pendant 2 jours à une température non permise (39,5 °C) avec 2 ml de MBE supplémenté avec 5 % SNV.

Ce procédé permet un enrichissement en virus de génome sauvage (Wt) ou recombinant.

Dans une seconde étape, les virus TK⁻ (recombinants) peuvent être sélectionnés comme indiqué précédemment en présence de 5BUdR à 37 °C. Dans ces conditions, les virus recombinants tk⁻ sont capables de se répliquer mais la croissance des virus Wt est empêchée par incorporation dans leur ADN d'analogue de dTTP.

Les virus sont clonés dans une première étape en utilisant un milieu solide contenant 1 % d'agar, des cellules LMTK⁻, 5 % de SNV (Drillien et coll., 1982) et reclonés à nouveau sur des cellules TK⁻ en présence de 5BUdR. Pour vérifier la recombinaison correcte du facteur IX dans le gène tk de VV, l'ADN total des cellules CEF infectées avec le virus recombinant est isolé et digéré avec diverses endonucléases. Après fractionnement, l'ADN est transféré sur filtres de nitrocellulose et hybridé avec un cADN de facteur IX marqué au phosphore radioactif avec l'ADN polymérase de E. coli.

On sélectionne ainsi un virus VVRTG2 utilisé dans les expériences ci-après.

Le virus isolé est ensuite cultivé sur des tapis de cellules CEF pour obtenir une grande quantité de virus en vue des expériences suivantes.

Infection des cellules de la lignée HepG2 par le virus de la vaccine

Les cellules HepG2 sont cultivées jusqu'à confluence dans des bouteilles à fond plat contenant un milieu composé de MEM (MEM,GIBCO) et de 5 % de sérum de veau fœtal (SVF) (GIBCO) et supplémenté avec de la vitamine K (50 µg/ml). Ces boîtes contiennent environ $10^7$ cellules.

Elles sont infectées soit avec le virus de la vaccine recombinant, soit avec le virus sauvage. Pratiquement le milieu de culture des cellules est retiré et 500 µl d'un extrait contenant le virus de la vaccine sont déposés sur les cellules (soit environ 5 pfu/cellule). Les boîtes sont agitées à la température de la pièce pendant 1 heure, puis 10 ml de MEM additionné de SVF (5 %) et de vitamine K (50 µg/ml) sont ajoutés. Les boîtes sont placées dans un incubateur à 97 °C pendant différents temps (4 heures, 8 heures, 22 heures).

Analyse de milieux de culture des cellules infectées

On a étudié les surnageants de cellules hépatiques infectées par le virus de la vaccine sauvage et le virus recombinant. Cette analyse a été accomplie par l'étude des milieux 4 heures, 8 heures et 22 heures après l'infection. Les milieux ont été fractionnés par adsorption du facteur IX sur le citrate de barium selon un procédé classique (dérivé de Miletrich et coll.) : le facteur IX peut s'adsorber sur ce sel insoluble quand les acides carboxylés sont présents sur la molécule.

Pratiquement à un volume de surnageant de cellules infectées (7 ml de surnageant de cellules infectées par le virus de la vaccine recombinant ou normal) on ajoute du citrate de sodium (30 mM final) puis on introduit au goutte à goutte 1/10 de volume d'une solution de chlorure de barium 1 M tout en agitant continuellement.

On laisse agiter pendant une heure le mélange. Le précipité est centrifugé à 8 000 g pendant 20 minutes.

Le surnageant est récolté et subit un fractionnement de 30 à 70 % de sulfate d'ammonium. La fraction précipitée avec 70 % de $NH_2SO_4$ est reprise par addition de 200 µl de Tris 20 mM NaCl 150 mM, pH 7,5, et est dialysé contre ce même tampon. Le volume final est alors de 800 µl environ. Le précipité de barium est resuspendu dans 0,3 volume de $BaCl_2$ 10 mM, NaCl 100 mM, puis recentrifugé à 8 000 g pendant 10 minutes. Cette opération de lavage du culot est répétée encore deux fois. Ensuite, le culot est repris dans 0,3 volume de Tris 20 mM, NaCl 150 mM, pH 7,5, puis on ajoute au goutte à goutte 0,075 volume de sulfate d'ammonium 2 M. Le mélange est agité pendant 1 heure puis centrifugé à 8 000 g 10′. Les protéines du surnageant sont précipitées du sulfate d'ammonium (concentration 70 %) pendant 1 heure. Les protéines précipitées sont centrifugées 10′ à 8 000 g.

Le culot est dissous dans 200 µl de Tris 20 mM NaCl 150 mM, pH 7,5 et est dialysé contre le même tampon.

L'ensemble de ces opérations est accompli à 0 °C.

Les fractions obtenues sont ensuite conservées à — 20 °C.

Elles sont ensuite testées de deux façons :

a) La quantité d'antigène facteur IX est estimée avec un test ELISA (commercialisé par Diagnostica-Stago).

b) L'activité du facteur est calculée en mesurant le temps de coagulation d'un plasma d'hémophile supplémenté avec les extraits.

Ces mesures sont comparées avec celles obtenues en utilisant un mélange de plasmas humains normaux.

Les résultats s'expriment en pourcentage. Pratiquement 100 % correspond à la quantité de facteur présent dans 1 ml de sang humain normal.

Les résultats sont fournis sur le tableau joint, page 40. Dans ce tableau sont indiquées les concentrations en facteur IX par ml d'échantillon. Dans les surnageants des cellules hépatiques infectées avec les virus de la vaccine sauvage, aussi bien dans la fraction adsorbée sur le citrate de barium que sur la fraction non adsorbée, la quantité d'antigène du facteur IX est inférieure à 1 %, l'activité coagulante reste inférieure à 2 %. En revanche dans les surnageants des cellules infectées par le virus recombinant, la quantité de facteur IX dans la fraction adsorbable sur le citrate de barium augmente avec le temps d'infection. Les quantités estimées par les tests ELISA et d'activité sont semblables. Dans la fraction non adsorbée sur le citrate de barium la quantité d'antigène reste faible (≤ 5 %) et l'activité reste comparable au bruit de fond détecté avec le virus sauvage.

En conclusion, les cellules HepG2 infectées avec le virus de la vaccine recombinant produisent du facteur IX actif. L'absence d'antigène facteur IX dans la fraction non adsorbée sur le citrate de barium montre que le facteur IX produit dans ces conditions est γ carboxylé pratiquement dans sa totalité.

Expression dans d'autres cellules

Les procédés expérimentaux sont similaires à ceux déjà décrits.

Ainsi, des tapis de cellules confluentes ont été infectés soit avec le virus sauvage soit avec le virus recombinant (1 à 5 pfu/cellule) dans les conditions décrites précédemment. L'infection de cellules (c'est-

## EP 0 162 782 B1

à-dire la multiplication du virus dans les cellules) a été accomplie 22 heures dans les milieux de culture MEM (Chang), Dulbecco (Vero), MEM-Galsgow (BHK21) supplémentés avec 10 % de sérum de veau fœtal. Ces milieux sont en outre additionnés de vitamine K (n° V3501, catalogue Sigma) à raison de 50 µg/ml.

Après l'infection, les milieux de culture ont été fractionnés comme décrit précédemment.

Le tableau A décrit les résultats obtenus. Ils sont exprimés en unité et non en pourcent comme décrit pour les cellules HepG2. Une unité représente la quantité de facteur IX dans 1 ml de plasma humain normal.

### Tableau A

Etude du facteur IX produit dans plusieurs
lignées infectées par VVRTG2

| Lignée | BHK21 | | Vero | | Chang | |
|---|---|---|---|---|---|---|
| Virus | WT : | CII | WT : | CII | WT : | CII |
| Volume initial milieu de culture | 53 | 55 | 40 | 40 | 30 | 30 |
| Volume fraction non adsorbée sur Barium (FI) (ml) | 5 | 5 | 3,6 | 3,8 | 7 | 5 |
| Volume fraction absorbée sur Barium (FII) (ml) | 0,6 | 0,5 | 0,65 | 0,40 | 0,45 | 0,26 |
| Quantité antigène dans FI (U) | 0,05 | 1,45 | $\leqslant$ 0,02 | 0,15 | $\leqslant$ 0,07 | 0,10 |
| Quantité antigène dans FII (U) | 0,02 | 3,65 | $\leqslant$ 0,02 | 1,09 | $\leqslant$ 0,02 | 0,58 |
| Quantité activité dans FII (U) | 0,01 | 4,55 | $\leqslant$ 0,02 | 1,07 | 0,16 | 0,73 |

Les résultats montrent que les trois lignées cellulaires infectées par le virus recombinant peuvent produire du facteur IX actif.

Cas des cellules HepG2

Les cellules HepG2 ont été cultivées pendant 12 à 15 générations (4 passages) dans du milieu MEM supplémenté avec 1 % de substitut de sérum synthétique Ultroser G, commercialisé par IBF (n° catalogue 50902), ce milieu était supplémenté ou pas avec la vitamine K. Les cellules ont ensuite été infectées avec les virus. Les résultats obtenus sont rassemblés au tableau B.

Tableau B

Effet de la vitamine K sur la synthèse du facteur IX
dans les cellules HepG2 infectées par VVRTG2

| Virus | VVRTG2 | | WT | | VVRTG2 | |
|---|---|---|---|---|---|---|
| Sérum | Ultroser G | | Ultroser G | | SVF | |
| Vitamine K | + | − | + | − | + | − |
| Volume milieu culture (ml) | 10 | 10 | 10 | 10 | 10 | 10 |
| Quantité Ag fraction non adsorbée sur barium (U) | 0,02 | 0,26 | ⩽0,01 | ⩽0,01 | 0,08 | 0,92 |
| Quantité Ag fraction adsorbée sur barium (U) | 1,54 | 0,98 | ⩽0,02 | ⩽0,02 | 1,8 | 1,6 |
| Activité fraction adsorbée sur barium (U) | 1,32 | 0,36 | 0,04 | 0,04 | − | − |

Ce tableau B montre que les cellules cultivées et infectées en présence de vitamine K produisent du facteur IX actif γ carboxylé.

En revanche, les cellules cultivées et infectées sans supplément de vitamine K produisent un facteur IX moins γ carboxylé : la fraction non absorbée sur barium contient une quantité importante d'antigène facteur IX. De plus l'activité coagulante dans la fraction absorbée sur le barium est très inférieure au niveau attendu compte tenu de la quantité d'antigène.

Un effet similaire est noté lorsque les cellules sont cultivées dans le milieu normal : si la vitamine K est omise au cours de l'infection la fraction non adsorbée sur le barium contient notablement plus d'antigène facteur IX.

Effet sur les cellules Vero

Les cellules Vero cultivées dans le milieu Dulbecco, additionné de sérum de veau fœtal (10 %) ont été infectées avec le virus VVRTG2 ou sauvage. La multiplication des virus a été accomplie dans ce même milieu, éventuellement supplémenté en vitamine K. Les résultats obtenus sont rassemblés au tableau C.

Tableau C

Effet de la vitamine K sur la synthèse du facteur IX
dans les cellules Vero infectées avec le virus VVRTG2

| Virus | VVRTG2 | | | WT | VVRTG2 |
|---|---|---|---|---|---|
| Temps d'infection | 12 | 22 | 38 | 22 | 22 |
| Vitamine K (supplément) | − | − | − | + | + |
| Volume milieu de culture (ml) | 20 | 20 | 10 | 40 | 40 |
| Quantité Ag fraction non adsorbée sur barium (U) | 0,86 | 1,06 | 0,73 | 0,04 | 0,30 |
| Quantité Ag fraction adsorbée sur barium (U) | 0,18 | 0,20 | 0,22 | ⩽0,01 | 1,09 |
| Activité fraction absorbée sur barium (U) | 0,21 | 0,08 | 0,08 | ⩽0,02 | 1,07 |

Le tableau C montre que l'addition de vitamine K dans le milieu de culture est très importante pour l'activité du facteur IX produit. On peut noter que l'importance de ce supplément de vitamine est d'autant plus important que le temps d'infection est long.

L'ensemble de ces résultats montre l'importance du supplément de vitamine K dans les milieux de culture des cellules infectées.

Fractionnement du facteur IX produit par les cellules infectées par le virus vaccine sauvage (WT) ou recombinant (CII)

Les différentes lignées de cellules animales ont été infectées par le virus recombinant ou sauvage à une multiplicité de 1 pfu/cellule. Après 24 heures, les milieux de culture sont récoltés puis fractionnés sur citrate de barium. Le facteur IX est ensuite dosé dans les deux fractions au moyen d'un test ELISA, seule la fraction adsorbée sur barium est dosée avec un test d'activité (mesure de la restauration de la coagulation d'un plasma d'hémophile).

Résultats exprimés en unité de facteur IX :

Tableau D

| Lignées | Virus | Quantité de facteur IX avant fractionnement (ELISA) | Quantité de facteur IX après fractionnement | | |
|---------|-------|------|------|------|------|
| | | | Fraction non adsorbée sur barium (ELISA) | Fraction adsorbée sur barium (ELISA) | (ACTIVITE) |
| Namalwa | CII | 14,5 | 0,42 | 0,41 | 0,50 |
| | WT | 0,5 | 0,06 | 0,03 | 0,30 |
| 143B | CII | 3,5 | 0,48 | 0,92 | 0,40 |
| | WT | 0,5 | 0,08 | 0,04 | 0,08 |
| MRC5 | CII | 4,0 | 0,70 | 0,34 | 0,48 |
| | WT | 0,5 | 0,07 | 0,04 | 0,36 |
| MDCK | CII | 7,0 | 0,98 | 0,88 | 0,24 |
| | WT | 0,5 | 0,08 | 0,04 | 0,04 |
| LM-TK⁻ | CII | – | 0,18 | 0,66 | 0,18 |
| | WT | 0,4 | 0,01 | 0,01 | 0,02 |
| CEF* | CII | 12 | 2,94 | 1,96 | 4,16 |
| | WT | 0,5 | 0,07 | 0,04 | 1,92 |

\* cellules embryonnaires de poulets

Ces résultats montrent que toutes les cellules testées sont capables d'opérer la γ-carboxylation du facteur IX ; cependant, l'efficacité de cette modification post-traductionnelle varie suivant les lignées. Ces résultats démontrent que les cellules en cultures testées peuvent être utilisées pour produire des protéines γ-carboxylées et possédant une activité biologique : le facteur IX récupéré dans les milieux de culture est au moins partiellement actif.

Cellules primaires de rein de singe

Les cellules de rein de singe récemment trypsiné ont été placées en culture. Cette culture primaire une fois confluente a été infectée dans les conditions décrites ci-dessus.

Après 24 heures d'infection, les milieux de culture ont été collectés et fractionnés sur barium.

Les résultats sont les suivants (pour 40 ml de milieu) :

# EP 0 162 782 B1

Tableau E

Ces résultats montrent que les cellules primaires de rein de singe peuvent être utilisées pour la production de facteur IX actif.

|  | Facteur IX dans le milieu brut (U antigène) | Facteur IX dans la fraction non adsorbée (U antigène) | Facteur IX dans la fraction adsorbée (U antigène) | Facteur IX dans la fraction adsorbée (U activité) |
|---|---|---|---|---|
| Virus WT | < 0,4 | < 0,08 | < 0,02 | 0,24 |
| Virus CII | 12,4 | 1,8 | 1,9 | 2,0 |

Expression du facteur IX en utilisant le virus Cowpox

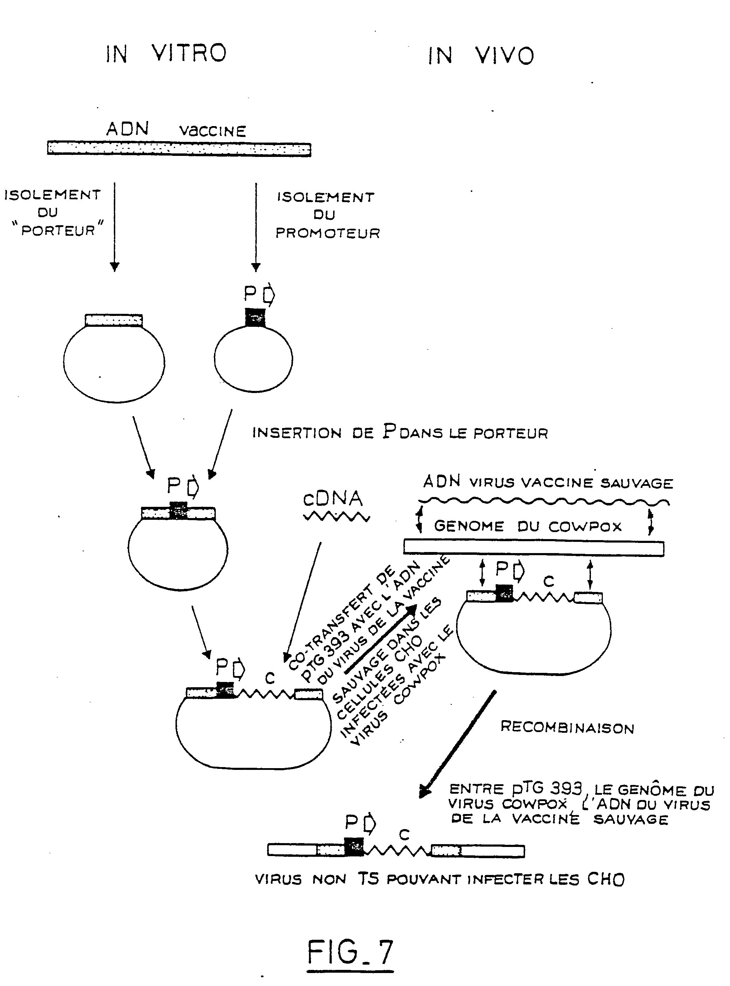

Le vecteur est construit en utilisant le schéma expérimental (figure 7) déjà décrit pour l'isolement de VVRTG2. Quelques modifications du protocole expérimental sont toutefois nécessaires.

Les cellules CHO-K1 (ATCC CCL61) ont été utilisées à la place des cellules embryonnaires de poulets. Pour l'infection, le virus du Cowpox, souche Brighton, a été employé. Les milieux de culture utilisés sont ceux préconisés pour les cellules CHO : le MBE a été substitué par le milieu Alpha 2000 et le sérum SVN a été remplacé par le sérum de veau fœtal (SVF).

La construction est identique jusqu'à la construction de pTG393.

On effectue ensuite un cotransfert entre pTG393, l'ADN du virus de la vaccine sauvage dans les cellules CHO infectées avec le virus Cowpox, comme décrit précédemment. Il convient de remarquer que si dans la construction de VVRTG2 la recombinaison entre l'ADN du plasmide, l'ADN de la vaccine cotransféré et le génome viral n'est pas établie ou comprise, le protocole utilisé pour le recombinant Cowpox requiert la recombinaison des 3 entités.

Le virus obtenu après recombinaison est non ts et peut infecter les cellules CHO.

Le procédé utilisé pour l'expérience de recombinaison permet d'enrichir la production de virus en virus recombinants entre le génome du virus Cowpox (thermosensible sur les cellules CHO) et l'ADN du virus de la vaccine (non thermosensible) et, éventuellement, le vecteur du facteur IX, pTG393.

Les virus produits sont ensuite clonés et sélectionnés pour leur caractère TK⁻ comme indiqué précédemment.

On a ensuite vérifié que le cADN du facteur IX est bien intégré dans le génome viral. Un clone sélectionné a été utilisé pour l'étude de l'expression du facteur IX dans les cellules CHO.

Pour cela, les cellules CHO-K1 sont infectées à 1 pfu par cellule et l'infection accomplie dans le milieu Alpha 2000 supplémenté en SVF et vitamine K. Le milieu de culture est fractionné sur citrate de barium.

Un résultat typique est le suivant :

Pour 30 ml de culture, on obtient 6,4 U d'antigène :

dans la fraction non absorbée, on récolte 0,3 U d'antigène,

dans la fraction absorbée, 0,86 U d'antigène et 0,64 U d'activité coagulante.

Le facteur IX produit dans les cellules CHO est actif.

Dans la fraction absorbée sur barium, il existe une différence de 20 % entre l'activité mesurée et l'activité attendue compte tenu de la quantité d'antigène. Cette différence peut être significative, mais d'autres tests plus précis devraient être accomplis pour déterminer si le facteur IX produit par les cellules CHO est complètement actif. On peut cependant affirmer que le facteur IX produit dans les cellules CHO possède une activité voisine de celle de la molécule humaine naturelle.

La souche suivante a été déposée à la Collection Nationale de Cultures de Microorganismes de l'INSTITUT PASTEUR, 28 rue du Docteur-Roux, PARIS 15ème le 30 avril 1985 : E. coli, comportant le plasmide pTG393, sous le n° 1-446.

Analyse des milieux de culture de cellule hépatique HepG2 infectés par le virus de
la vaccine de type sauvage ou recombinant avec pTG393.

| | | Virus de type sauvage | | | Virus recombinant | | |
|---|---|---|---|---|---|---|---|
| | | Temps d'infection | | | Temps d'infection | | |
| | | 4h | 8h | 22h | 4h | 8h | 22h |
| Fraction non adsorbée | Quantité antigène (%/ml) | 0,36 | 0,33 | 0,95 | 4,2 | 5,57 | 1,84 |
| au citrate de Barium | Activité coagulante (%/ml) | 2,3 | 1,7 | 1,7 | 1,7 | 1,7 | 1,8 |
| Fraction adsorbée sur | Quantité antigène (%/ml) | 0,29 | 0,41 | 0,559 | 1,0 | 4,2 | 89,3 |
| le citrate de barium | Activité coagulante (%/ml) | 1,2 | 1,2 | 0,9 | 3,3 | 5,8 | 71,2 |

EP 0 162 782 B1

## Bibliographie

Bertina R.M. et Veltkamp J.J. (1981) In Hemostasis and Thrombosis, Bloom A.L. et Thomas D.T. Eds. pp. 98-110, Churchill Livingstone, London.

Hedner U. et Davie E.W. (1982), in Hemostasis and Thrombosis, Colman R.W., Hirsh J., Marder V.J. et Salzman E.W. Eds. pp. 29-38, J.B. Lippincott Co., Philadelphia.

Tolstoshev P., Berg R.A., Rennard S.I., Bradley K.N., Trapnel B.C. et Crystal R.G. (1981). J. Biol. Chem. 256, 3135-3140.

Deng G. et Wu R. (1981). Nucl. Acids Res. 9, 4173-4188.

Murray N.E., Brammar W.J. et Murray K. (1977), Mol. Gen. Genetics 150, 53-61.

MacGillivray R.T.A., Degen S.H.F., Chandra T., Woo S.L.C. et Davie E.W. (1980). Proc. Natl. Acad. Sci. USA, 77, 5153-5157.

Chung D.W., Rixon M.W., MacGillivray R.T.A. et Davie E.W. (1981). Proc. Natl. Acad. Sci. USA 78, 1466-1470.

Kohli V., Balland A., Wintzerith M., Sauerwald R., Staub A. et Lecocq J.P. (1982). Nucl. Acids Res. 10, 7439-7448.

Fritz H.J., Belagaje R., Brown E.L., Fritz R.H., Jones R.A., Lees R.F. et Khorana H.G. (1978). Biochemistry 17, 1257-1267.

Grunstein M. et Wallis J. (1979). Methods in Enzymology 68 ; 379-389.

Katayama K., Ericsson L.H., Enfiled D.L., Walsh K.A., Neurath H., Davie E.W. et Titani K. (1979). Proc. Natl. Acad. Sci. USA 76, 4990-4994.

Di Scipio R.G., Hermodson M.A., Yates S.G. et Davie E.W. (1977). Biochemistry 16, 698-706.

Messing J. et Vieira J. (1982). Gene 19, 269-276.

Sanger F., Nicklen S. et Coulson A.R. (1977) ; Proc. Natl. Acad. Sci. USA, 74, 5463-5467.

Maxam A.M. et Gilbert W. (1980). Methods in Enzymology 65, 499-555.

Di Scipio R.G., Kurachi K. et Davie E.W. (1978). J. Clin. Inves. 61, 1528-1538.

Cooper D.W. (1978) in The Biochemical Genetics of Man, Brock D.J.H. et Mayo O., pp. 271-324, Eds. Academic Press London.

Mizuochi T., Taniguchi T., Fujikawa K., Titani K. et Kobata A. (1983). J. Biol. Chem. 258, 6020-6024.

Drillien R. et Spehner D. (1983) Virology 131, 385-393.

Kieny M.P., Lathe R. et Lecocq J.P. (1983) Gene 26, 91-99.

Lathe R., Kieny M.P., Skory S. et Lecocq J.P. (1984), DNA, in press.

Lusky M., Botchan M. (1981). Nature 293, 79-81.

Mackett M., Smith J.L. & Moss B. (1982). Proc. Natl. Acad. Sci. USA 79, 7415-7419.

Panicali D. & Paoletti E. (1982). Proc. Natl. Acad. Sci. USA 79, 4927-4931.

Panicali D., Davis S.W., Weinberg R.L. & Paoletti E. (1983). Proc. Natl. Acad. Sci. USA 80, 5364-5368.

Smith G.L., Mackett M. et Moss B. (1983). Nature 302, 490-495.

Venkatesan S., Baroudy B.M. & Moss B. (1981). Cell 125, 805-813.

Vieira J. & Messing J. (1982). Gene 18, 259-268.

Weir J.P. & Moss B. (1983). J. Virol. 46, 530-537.

Drillien R., Spehner D. & Kirn A. (1982). Virology 119, 372-381.

Drillien R., Koehren F. & Kirn A. (1981). Virology 111, 488-499.

Kurachi K., Davie E.W. (1982). Proc. Natl. Acad. Sci., USA 79, 6461-6464.

Miletrich J.P., Broze G.J., Majerus P.W. (1981). Methods in Enzymology 80, 221-228 (Academic Press Inc.).

Jaye M., de la Salle H., Schamber F., Balland A., Kohli V., Findeli A., Tolstoshev P., Lecocq J.P. (1983) Nucl. Acids Res., 11, 2325-2335.

Southern E.M. (1975). J. Mol. Biol., 94, 51-69.

## Revendications

1. Procédé de préparation du facteur IX ou d'une protéine de structure analogue, caractérisé en ce que l'on cultive des cellules infectées par un vecteur d'expression du facteur IX constitué du génome d'un virus de la famille des Poxvirus dans lequel a été inséré un gène codant pour le facteur IX humain ou une protéine analogue du facteur IX, et en ce que le milieu de culture comporte de la vitamine K et en ce que l'on récupère, après culture, le facteur IX ou la protéine de structure analogue.

2. Procédé selon la revendication 1, caractérisé en ce que les cellules infectées sont choisies parmi les cellules hépatiques, les cellules Vero, BHK et CHO et les sous-clones de ces lignées.

3. Procédé selon l'une des revendications 1 et 2, caractérisé en ce que la quantité de vitamine K ajoutée est telle qu'elle assure la saturation du milieu de culture.

4. Procédé selon l'une des revendications 1 à 3, caractérisé en ce que le Poxvirus est du genre Orthopoxvirus.

5. Procédé selon la revendication 4, caractérisé en ce que l'Orthopoxvirus est choisi parmi la vaccine et le virus Cowpox.

6. Procédé selon l'une des revendications 1 à 5, caractérisé en ce que le gène est sous le contrôle d'un promoteur de Poxvirus.

7. Procédé selon la revendication 6, caractérisé en ce que ledit gène est sous la promotion d'un promoteur de la vaccine.

8. Procédé selon la revendication 7, caractérisé en ce que ledit promoteur de la vaccine est le promoteur du gène de la protéine 7,5K.

9. Procédé selon l'une des revendications 4 à 8, caractérisé en ce que ledit gène est cloné dans le gène TK de la vaccine.

## Claims

1. A method of preparing factor IX or a proteub of similar structure, characterised by cultivation of cells infected with a vector for expressing the factor IX and comprising the genome of a virus of the Poxvirus family into which a gene which codes for human factor IX or a protein similar to factor IX has been inserted, and the culture medium comprises vitamin K, and the factor IX or the protein of similar structure is recovered after cultivation.

2. A method according to claim 1, characterised in that the infected cells are chosen from among liver cells, Vero, BHK and CHO cells and sub-clones of these strains.

3. A method according to claim 1 or 2, characterised in that the quantity of vitamin K added is sufficient to saturate the culture medium.

4. A method according to any of claims 1 to 3, characterised in that the Poxvirus is of the genus Orthopoxvirus.

5. A method according to claim 4, characterised in that the Orthopoxvirus is chosen from among Cowpox virus and vaccine.

6. A method according to any of claims 1 to 5, characterised in that the gene is under the control of a Poxvirus promoter.

7. A method according to claim 6, characterised in that the gene is promoted by a promoter of the vaccine.

8. A method according to claim 7, characterised in that the vaccine promoter is the promoter of the gene of protein 7,5K.

9. A method according to any of claims 4 to 8, characterised in that the gene is cloned in the TK gene of the vaccine.

## Patentansprüche

1. Verfahren zur Herstellung des Faktors IX oder eines Proteins mit analoger Struktur, dadurch gekennzeichnet, daß man Zellen kultiviert, die mit einem Expressionsvektor des Faktors IX, bestehend aus dem Genom eines Virus der Familie der Poxviren, in das ein Gen inseriert worden ist, das für den Human-Faktor IX oder ein zum Faktor IX analoges Proteins kodiert, infiziert worden sind, und daß das Kulturmilieu Vitamin K enthält und daß man nach der Kultivierung den Faktor IX oder das Protein mit analoger Struktur gewinnt.

2. Verfahren nach Anspruch 1, dadurch gekennzeichnet, daß die infizierten Zellen ausgewählt werden aus Leberzellen, Vero-, BHK- und CHO-Zellen und den Subklonen dieser Zellinien.

3. Verfahren nach den Ansprüchen 1 und 2, dadurch gekennzeichnet, daß die zugegebene Menge an Vitamin K so groß ist, daß die Sättigung des Kulturmediums gewährleistet ist.

4. Verfahren nach einem der Ansprüche 1 bis 3, dadurch gekennzeichnet, daß das Poxvirus ein solches vom Genus Orthopoxvirus ist.

5. Verfahren nach Anspruch 4, dadurch gekennzeichnet, daß das Orthopoxvirus ausgewählt wird aus dem Vakzin und dem Virus Cowpox.

6. Verfahren nach einem der Ansprüche 1 bis 5, dadurch gekennzeichnet, daß das Gen unter der Kontrolle eines Promotors für Poxvirus steht.

7. Verfahren nach Anspruch 6, dadurch gekennzeichnet, daß das Gen unter der Promotion eines Promoters für das Vakzin steht.

8. Verfahren nach Anspruch 7, dadurch gekennzeichnet, daß es sich bei dem Promotor für das Vakzin um den Promotor für das Gen des Proteins 7,5K handelt.

9. Verfahren nach einem der Ansprüche 4 bis 8, dadurch gekennzeichnet, daß das Gen in das Gen TK des Vakzins kloniert ist.

EP 0 162 782 B1

       35                     40              45            50

a.   thr glu lys thr thr glu phe trp lys gln tyr val asp gly asp gln cys glu ser

b.   ACX GA$_G^A$ AA$_G^A$ ACX ACXGA$_G^A$ TT$_T^C$ TGG AA$_G^A$ CA$_G^A$ TA$_T^C$ GTX GA$_T^C$ GGX GA$_T^C$ CA$_G^A$ TG$_T^C$ GA$_G^A$ TCX
                                                                                       AG$^C$

c.   T GAG AAGACT ACA GAG TTC TGG AAG CAG TAT GTG GAT GGT GAT CAG TGT GAG
    + ++ +    + +  ++  ++  ++  +++ +.+++++ +++ ++  +++ ++  +++ +++ +++ +++

d.   TGAA AGAACA ACTGAA TTT TGGAAG CAG TAT GTT GAT GGAGAT CAGTGT GAG
   244      250          260         270         280         290

## FIG_1

```
1                           25                          50                          75
T ATG CAG CGC GTG AAC ATC ATC ATG GCA GAA TCA CCA GGC CTC ATC ACC ATC TGC CTT TTA GGA TAT CTA CTC AGT
  met gln arg val asn met ile met ala glu ser pro gly leu ile thr ile cys leu leu gly tyr leu leu ser

                            100                         125                         150
GCT GAA TGT ACA GTT TTT CTT GAT CAT GAA AAC GCC AAC AAA ATT CTG AAT CGG CCA AAG AGG TAT AAT TCA GCT
ala glu cys thr val phe leu asp his glu asn ala asn lys ile leu asn arg pro lys arg tyr asn ser gly

                            175                         200                         225
AAA TTG GAA GAG TTT GTT CAA GGC AAC CTT GAG AGA GAA TGT ATG GAA GAA AAG TGT AGT TTT GAA GAA GCA CGA
lys leu glu glu phe val gln gly asn leu glu arg glu cys met glu glu lys cys ser phe glu glu ala arg

                            250                         275                         300
GAA GTT TTT GAA AAC ACT GAA AGA ACA ACT GAA TTT TGG AAG CAG TAT GTT GAT GGA GAT CAG TGT GAG TCC AAT
glu val phe glu asn thr glu arg thr thr glu phe trp lys gln tyr val asp gly asp gln cys glu ser asn

                            325                         350                         375
CCA TGT TTA AAT GGC GGC AGT TGC AAG GAT GAC ATT AAT TCC TAT GAA TGT TGG TGT CCC TTT GGA TTT GAA GGA
pro cys leu asn gly gly ser cys lys asp asp ile asn ser tyr glu cys trp cys pro phe gly phe glu gly

                            400                         425                         450
AAG AAC TGT GAA TTA GAT GTA ACA TGT AAC ATT AAG AAT GGC AGA TGC GAG CAG TTT TGT AAA AAT AGT GCT GAT
lys asn cys glu leu asp val thr cys asn ile lys asn gly arg cys glu gln phe cys lys asn ser ala asp

                            475                         500                         525
AAC AAG GTC CTT TGC TCC TGT ACT GAG GGA TAT CGA CTT GCA GAA AAC CAG AAG TCC TGT GAA CCA GCA GTG CCA
asn lys val val cys ser cys thr glu gly tyr arg leu ala glu asn gln lys ser cys glu pro ala val pro

                            550                         575                         600
TTT CCA TGT GGA AGA GTT TCT GTT TCA CAA ACT TCT AAG CTC ACC CGT GCT GAG ACT GTT TTT CCT GAT GTG GAC
phe pro cys gly arg val ser val ser gln thr ser lys leu thr arg ala glu thr val phe pro asp val asp

                            625                         650                         675
TAT GTA AAT TCT ACT GAA GCT GAA ACC ATT TTG GAT AAC ATC ACT CAA AGC ACC CAA TCA TTT AAT GAC TTC ACT
tyr val asn ser thr glu ala glu thr ile leu asp asn ile thr gln ser thr gln ser phe asn asp phe thr

                            700                         725                         750
CGG GTT GTT GGT GGA GAA GAT GCC AAA CCA GGT CAA TTC CCT TGG CAG GTT GTT TTG AAT GGT AAA GTT GAT GCA
arg val val gly gly glu asp ala lys pro gly gln phe pro trp gln val val leu asn gly lys val asp ala

                            775                         800                         825
TTC TGT GGA GGC TCT ATC GTT AAT GAA AAA TGG ATT GTA ACT GCT GCC CAC TGT GTT GAA ACT GGT GTT AAA ATT
phe cys gly gly ser ile val asn glu lys trp ile val thr ala ala his cys val glu thr gly val lys ile

                            850                         875                         900
ACA GTT GTC GCA GGT GAA CAT AAT ATT GAG GAG ACA GAA CAT ACA GAG CAA AAG CGA AAT GTG ATT CGA ATT ATT
thr val val ala gly glu his asn ile glu glu thr glu his thr glu gln lys arg asn val ile arg ile ile

                            925                         950                         975
CCT CAC CAC AAC TAC AAT GCA GCT ATT AAT AAG TAC AAC CAT GAC ATT GCA CTT CTG GAA CTG GAC GAA CCC TTA
pro his his asn tyr asn ala ala ile asn lys tyr asn his asp ile ala leu leu glu leu asp glu pro leu

                            1000                        1025                        1050
GTG CTA AAC AGC TAC GTT ACA CCT ATT TGC ATT GCT GAC AAG GAA TAC ACG AAC ATC TTC CTC AAA TTT GGA TCT
val leu asn ser tyr val thr pro ile cys ile ala asp lys glu tyr thr asn ile phe leu lys phe gly ser

                            1075                        1100                        1125
GGC TAT GTA AGT GGC TGG GGA AGA GTC TTC CAC AAA GGG AGA TCA GCT TTA GTT CTT CAG TAC CTT AGA GTT CCA
gly tyr val ser gly trp gly arg val phe his lys gly arg ser ala leu val leu gln tyr leu arg val pro

                            1150                        1175                        1200
CTT GTT GAC CCA GCC ACA TGT CTT CGA TCT ACA AAG TTC ACC ATC TAT AAC AAC ATG TTC TGT GCT GGC TTC CAT
leu val asp pro ala thr cys leu arg ser thr lys phe thr ile tyr asn asn met phe cys ala gly phe his

                            1225                        1250                        1275
GAA GGA GGT AGA GAT TCA TGT CAA GGA GAT ACT GGG GGA CCC CAT GTT ACT GAA GTG GAA GGG ACC AGT TTC TTA
glu gly gly arg asp ser cys gln gly asp thr gly gly pro his val thr glu val glu gly thr ser phe leu

                            1300                        1325                        1350
ACT GGA ATT ATT AGC TGG GGT GAA GAG TGT GCA ATG AAA GGC AAA TAT GGA ATA TAT ACC AAG GTA TCC CGG TAT
thr gly ile ile ser trp gly glu glu cys ala met lys gly lys tyr gly ile tyr thr lys val ser arg tyr

                            1375                        1400                        1425
GTC AAC TGG ATT AAG GAA AAA ACA AAG CTC ACT TAA TGA AAG ATG GAT TTC CAA GGT TAA TTC ATT GGA ATT GAA
val asn trp ile lys glu lys thr lys leu thr --- ---

                            1450                        1475                        1500
AAT TAA CAG GGC CTC TCA CTA ACT AAT CAC TTT CCC ATC TTT TGT TAG ATT TGA ATA TAT ACA TTC TAT GAT CAT

                            1525                        1550                        1575
TGC TTT TTC TCT TTA CAG GGG AGA ATT TCA TAT TTT ACC TGA GCA AAT TGA TTA GAA AAT GGA ACC ACT AGA GCA

                            1600                        1625
ATA TAA TCT GTT AGG AAA TTA CAG TCA TTT CTA AGG GCC CAG CCT TGA CAA ATT GTG AGT AAA ................
```

FIG. 2.

FIG.3

FIG_4

FIG_5

FIG.6

FIG_7